# FASCICULE DE BREVET EUROPEEN

(11) **EP 3 993 721 B1**
(45) Date de publication et mention de la délivrance du brevet: **16.04.2025**
(21) Numéro de dépôt: 20736694.9
(22) Date de dépôt: 03.07.2020
(51) Int. Cl.: A61B 17/66

(54) **ENSEMBLE COMPRENANT UN APPAREIL D'EXPANSION OSSEUSE, UN OUTIL D'ACTIVATION ET UN DISPOSITIF DE POSITIONEMENT**
SET INKLUSIVE KNOCHENEXPANSIONSVORRICHTUNG, AKTIVIERUNGSWERKZEUG UND POSITIONIERVORRICHTUNG
SET COMPRISING A BONE EXPANSION APPARATUS, AN ACTIVATION TOOL AND A POSITIONING DEVICE

(30) Priorité: 05.07.2019 FR 1907567
(43) Date de publication de la demande: 11.05.2022
(73) Titulaire: École Nationale Supérieure de Techniques Avancées, 91120 Palaiseau (FR); ASSISTANCE PUBLIQUE, HOPITAUX DE PARIS, 75004 Paris (FR); Université de Paris Cité, 75006 Paris (FR)
(72) Inventeur: KADLUB, Natacha, 75011 Paris (FR); BOISSON, Jean, 75011 Paris (FR); DALLARD, Jérémy, 94340 Joinville-le-Pont (FR); KOGANE, Nicolas, 75013 Paris (FR)
(74) Mandataire: LLR
(86) Numéro de dépôt international: PCT/EP2020/068900
(87) Numéro de publication internationale: WO 2021/004972

(56) Documents cités:
- WO-A1-2015/168175
- WO-A1-2017/097998
- US-A1- 2014 128 868

## Description

L'invention concerne le domaine des appareils d'expansion osseuse, tels que, par exemple, un distracteur à plaques ou un disjoncteur. L'invention concerne plus particulièrement un ensemble, tel que défini dans la revendication 1, comprenant un appareil d'expansion osseuse, un outil d'activation et un dispositif d'aide au positionnement de l'outil d'activation par rapport à l' appareil d'expansion osseuse qui lui est associé.

On connaît déjà dans l'état la technique, notamment d'après le document WO 2017097998 A1, un distracteur à plaques prévu pour opérer une distraction maxillo-faciale. Un tel distracteur à plaques est fixé sur un côté d'un os ou sur le côté d'os voisins d'un sujet et comprend un aimant rotatif à proximité d'une extrémité du distracteur.

Une mise en rotation de cet aimant, à partir d'un outil d'activation externe également porteur d'un aimant, positionné selon un axe en correspondance de l'axe de rotation de l'aimant du distracteur, permet d'opérer une distraction osseuse. Pour ce faire, la rotation de l'aimant du distracteur entraine l'écartement des plaques du distracteur par le biais d'un mécanisme de vis sans fin. La vis sans fin opère une conversion du mouvement de rotation de l'aimant du distracteur en un mouvement de translation d'une plaque du distracteur par rapport à l'autre plaque, ce qui entraîne un écartement des plaques.

L'ensemble de distraction osseuse décrit dans ce document comprend un outil d'activation externe au sujet porteur du distracteur, dont la mise en rotation permet la rotation de l'aimant du distracteur à plaques par couplage magnétique.

Cet outil est toutefois utilisé manuellement et le contrôle de la rotation de l'aimant du distracteur à plaques requiert alors une grande maîtrise et une grande vigilance de l'opérateur.

L'opérateur doit alors être correctement formé pour ce type de manipulation.

Une erreur dans la manipulation de l'outil d'activation peut entraîner une distraction osseuse trop faible ou, au contraire, trop importante, ou encore refermer une fracture chirurgicale utile au traitement.

Les documents EP 1272115 A1 et EP 3061614 A1 décrivent un dispositif de distraction chirurgical pour appliquer une force de tension ou d'extension de manière non invasive sur le squelette d'un patient ou sur un implant. L'activateur décrit dans ces documents présente une structure périphérique à un membre d'un patient. Le document WO 2015168175 A1 décrit un implant médical permettant l'ajustement à distance d'une dimension entre deux parties d'un corps, par un exemple un dispositif distracteur, ainsi qu'une commande à distance pour l'ajustement d'un implant médical. Le document US 2014128868 A1 décrit un dispositif et un procédé de repositionnement de structures osseuses.

L'invention a notamment pour but de faciliter le contrôle d'un dispositif d'expansion osseuse tel que, conformément à l'invention, un distracteur à plaques, dont l'allongement est commandé par la rotation d'un aimant couplé à une vis sans fin.

A cet effet l'invention a pour objet un ensemble comprenant un distracteur à plaques, un outil d'activation du distracteru à plaques et un dispositif d'aide au positionnement de l'outil d'activation, l'outil d'activation comprenant un élément magnétique solidaire d'un axe de rotation de l'outil d'activation, l'élément magnétique étant configuré pour interagir, coaxialement, avec un élément magnétique distant porté par l'appareil d'expansion osseuse, l'outil d'activation comprenant en outre un moteur électrique configuré pour opérer une rotation de l'axe de rotation, et un module de contrôle de la rotation de l'axe de rotation.

Il suffit dans ce cas de placer l'élément magnétique de l'outil d'activation et l'élément magnétique distant porté par l'appareil d'expansion osseuse en vis-à-vis, ou en d'autres termes en regard l'un de l'autre. De cette manière, avec une telle configuration coaxiale, la rotation de l'élément magnétique de l'outil d'activation autour d'un axe de rotation de l'élément magnétique provoque la rotation de l'élément magnétique distant porté par l'appareil d'expansion osseuse autour d'un axe de rotation de l'élément magnétique distant. Dans cette configuration coaxiale, l'axe de rotation de l'élément magnétique distant coïncide avec l'axe de rotation de l'élément magnétique de l'outil d'activation. Ainsi, la rotation de l'élément magnétique distant porté par l'appareil d'expansion osseuse est obtenue via l'interaction coaxiale entre l'élément magnétique de l'outil d'activation et l'élément magnétique distant porté par l'appareil d'expansion osseuse, sans générer de force d'arrachement de l'appareil d'expansion osseuse selon une direction perpendiculaire à l'axe de rotation de l'élément magnétique distant. Cela est particulièrement avantageux lorsque l'appareil d'expansion osseuse est un distracteur à plaques. En effet, un tel distracteur à plaques est souvent utilisé sur de très jeunes enfants et est par exemple fixé sur les os à l'aide de vis de courte longueur, par exemple de 5 mm de longueur, voire avec des vis résorbables en matière plastique. L'enlèvement du distracteur à plaques s'effectue par arrachement. Ainsi, la fixation du distracteur à plaques sur les os est relativement fragile et le réglage du distracteur à plaques ne doit pas provoquer de contraintes mécaniques susceptibles de provoquer un arrachement prématuré. C'est pourquoi l'intensité des contraintes mécaniques susceptibles de s'exercer sur la fixation des plaques aux os doit rester la plus faible possible pour permettre l'actionnement du distracteur à plaques en toute sécurité.

Suivant d'autres caractéristiques optionnelles de l'outil d'activation prises seules ou en combinaison :
- L'outil d'activation est configuré pour contrôler le sens de rotation de l'axe de rotation. Il est ainsi possible de procéder aussi bien à un écartement d'éléments de fixation de l'appareil d'expansion osseuse qu'à un rapprochement de ceux-ci. En outre, en cas de dépassement de la course souhaitée, il est possible de réduire l'écartement obtenu, avec la même précision.
- L'outil d'activation est configuré pour contrôler la vitesse de rotation de l'axe de rotation. De cette façon, il est possible d'obtenir une plus grande précision et un meilleur contrôle de la rotation de l'axe de rotation et donc de l'écartement ou du resserrement d'éléments mécaniques de l'appareil d'expansion osseuse.
- Le module de contrôle de l'outil d'activation comprend des moyens de comptage d'un nombre de portions de tours réalisées lors de la rotation de l'axe de rotation. Ainsi, l'opérateur peut s'affranchir d'avoir à effectuer lui-même un comptage de tours ou de portions de tours de l'axe de rotation de l'outil d'activation, ce qui réduit conséquemment le risque d'erreur.
- Le module de contrôle de l'outil d'activation comprend des moyens d'arrêt de la rotation de l'axe de rotation lorsque l'axe de rotation a opéré une rotation d'un nombre prédéterminé de tours, de sorte que, dès que le nombre de tours ou de portions de tours à opérer est atteint, la rotation de l'axe de rotation est interrompue, ce qui permet d'éviter tout risque de dépassement lié à un temps de réaction trop important de l'opérateur.
- Le module de contrôle de l'outil d'activation comprend des moyens de programmation d'un sens de rotation et d'un nombre de tours de l'axe de rotation à opérer selon ce sens de rotation.

Avantageusement, l'outil d'activation réalise un contrôle de la rotation de son axe de rotation de façon automatisée, ce qui là encore réduit conséquemment le risque d'erreur et facilite l'opération.
- L'outil d'activation comprend un capteur de champ magnétique, tel que par exemple un capteur à effet Hall, configuré pour mesurer une variation de champ magnétique émis par l'élément magnétique distant porté par l'appareil d'expansion osseuse lorsque l'élément magnétique distant est positionné dans le prolongement de l'axe de rotation de l'outil d'activation du côté de l'élément magnétique solidaire de l'axe de rotation. Il est ainsi possible de transformer des variations d'un champ magnétique détecté en une information représentative de la rotation d'un élément magnétique distant. Le capteur de champ magnétique délivre une tension dont la variation est représentative de la variation du champ magnétique détecté.
- L'outil d'activation comprend un module de mesure de champ magnétique configuré pour déterminer une rotation de l'élément magnétique distant d'au moins un nombre de tours prédéterminé autour de l'axe d'écartement de l'appareil d'expansion osseuse. Grâce à ce module, il est possible de déterminer la rotation d'un élément magnétique distant à partir d'une variation de la tension délivrée par le capteur de champ magnétique. Avantageusement, la rotation de l'axe de rotation de l'outil d'activation est alors contrôlée selon la rotation effective de l'élément magnétique distant de l'appareil d'expansion osseuse, et non uniquement selon la rotation de l'élément magnétique de l'outil d'activation, grâce à une analyse des variations de champ magnétique mesurées. Ainsi, en cas de mauvais couplage magnétique, et si un phénomène de glissement apparait entre la rotation de l'élément magnétique de l'outil et la rotation de l'élément magnétique de l'appareil d'expansion, l'expansion réellement effectuée est malgré tout contrôlée.
- L'élément magnétique de l'outil d'activation comprend un aimant ou un ensemble de bobines électromagnétiques configurées pour permettre la génération d'un champ magnétique tournant orienté perpendiculairement à l'axe de rotation. Par exemple, l'élément magnétique est un aimant permanent, de préférence magnétisé selon une direction transverse à l'axe de rotation de cet aimant permanent de l'outil d'activation. Par exemple, l'aimant permanent est cylindrique.
- L'élément magnétique distant porté par l'appareil d'expansion osseuse est un aimant permanent, de préférence magnétisé selon une direction transverse à l'axe de rotation de cet aimant permanent de l'appareil d'expansion osseuse. Par exemple, l'aimant permanent est cylindrique.

Ainsi, du fait des directions de magnétisation des deux aimants permanents, un effort d'attraction ou de répulsion entre ces deux aimants est réduit, par rapport à une configuration où les aimants interagissent selon la direction de leurs axes d'aimantation. On réduit ainsi les contraintes engendrées par le distracteur à plaque sur l'os, sur lequel le distracteur à plaques est fixé, ces contraintes étant susceptibles de provoquer l'arrachement du distracteur à plaques.
- L'élément magnétique de l'outil d'activation est configuré pour être entraîné par le moteur électrique, via l'axe de rotation, à basse vitesse de rotation, par exemple au maximum entre 12 et 30 tours par minute, de préférence à 18 tours par minute. Ainsi, cela évite de prévoir un réducteur dans l'appareil d'expansion osseuse, du fait de la basse vitesse de rotation de l'élément magnétique distant lorsqu'il est couplé à l'élément magnétique de l'outil d'activation.

La fourniture d'un appareil d'expansion osseuse et d'un outil d'activation associé, dans un même ensemble, permet d'optimiser les caractéristiques des éléments magnétiques de sorte à en optimiser le couplage magnétique.

L'invention concerne un ensemble tel que décrit ci-avant comprenant er outre un dispositif accessoire d'aide au positionnement de l'élément magnétique de l'outil d'activation par rapport à l'élément magnétique porté par l'appareil d'expansion osseuse.

Suivant d'autres caractéristiques de l'ensemble, le dispositif d'aide au positionnement de l'élément magnétique de l'outil d'activation comprend une feuille en matériau formable ou thermo-formable configurée pour être rapportée sur un sujet et comprend un guide de positionnement de l'élément magnétique.

### Brève description des figures

L'invention sera mieux comprise à la lecture de la description qui va suivre donnée uniquement à titre d'exemple et faite en se référant aux dessins annexés dans lesquels :
[Fig. 1] La figure 1 est une vue schématique d'un outil d'activation d'un appareil d'expansion osseuse, positionné en regard d'un distracteur à plaques.
[Fig. 2] La figure 2 est un schéma simplifié de l'outil d'activation de la figure 1.
[Fig. 3] La figure 3 est une vue schématique d'une première variante de l'outil d'activation de la figure 1.
[Fig. 4] La figure 4 est un schéma simplifié de l'outil d'activation représenté sur la figure 3.
[Fig. 5] La figure 5 est une vue schématique d'une seconde variante de l'outil d'activation de la figure 1.
[Fig. 6] La figure 6 est un schéma simplifié de l'outil d'activation représenté sur la figure 5.
[Fig. 7] La figure 7 est une vue schématique d'un outil d'activation d'un appareil d'expansion osseuse, positionné en regard d'un distracteur à plaques par le biais d'un dispositif accessoire d'aide au positionnement de l'outil par rapport à un distracteur à plaques porté par un sujet.
[Fig. 8] La figure 8 représente un dispositif d'aide au positionnement de l'outil d'activation, avant mise en forme.
[Fig. 9] La figure 9 illustre une variante de l'outil d'activation d'un appareil d'expansion osseuse déjà représenté sur la figure 1, positionné en regard d'un disjoncteur.
[Fig. 10] La figure 10 est une vue en perspective d'un dispositif d'aide au positionnement d'un équipement de type outil d'activation.
[Fig. 11] La figure 11 est une vue éclatée en perspective d'un élément de guidage de l'équipement outil d'activation représenté sur la figure 10.
[Fig. 12] La figure 12 est une vue similaire à la figure 11, selon une première variante.
[Fig. 13] La figure 13 est une vue similaire à la figure 11, selon une seconde variante.
[Fig. 14] La figure 14 illustre une utilisation du dispositif d'aide au positionnement de la figure 10 pour le positionnement d'un activateur pour un distracteur dans le corps d'un sujet.
[Fig. 15] La figure 15 est une vue en perspective d'un dispositif d'aide au positionnement comprenant l'élément de guidage de la figure 13.
[Fig. 16] La figure 16 est une vue en perspective du dispositif d'aide au positionnement représenté sur la figure 15, sur lequel est positionnée l'extrémité d'une sonde.

### Description détaillée

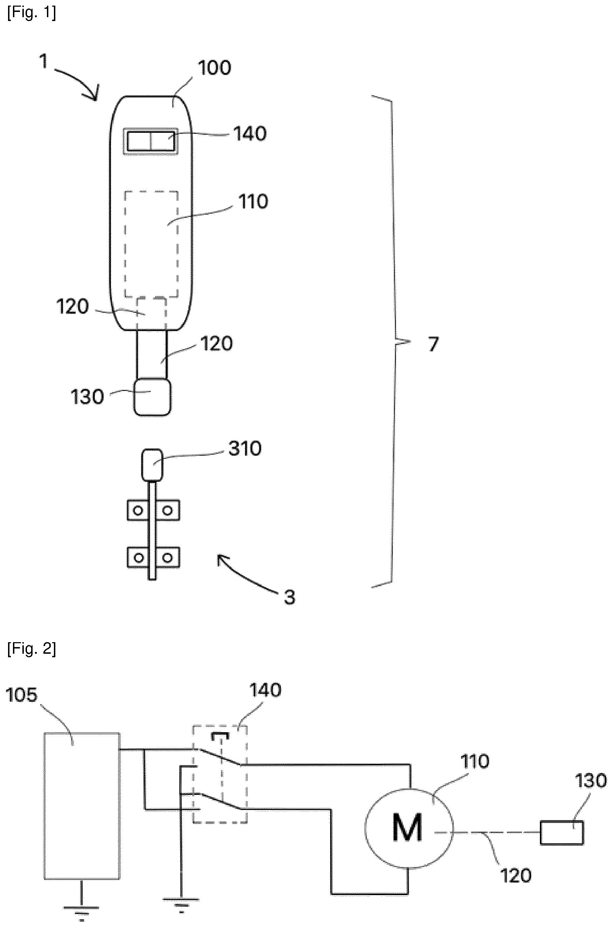

On a représenté sur les figures 1 à 7 et 9 un outil d'activation 1 pour un appareil d'expansion osseuse, notamment pour un distracteur à plaques 3 ou un disjoncteur 3', respectivement prévus pour allonger ou disjoindre des parties osseuses. Le distracteur à plaques 3 est représenté schématiquement, et est par exemple un distracteur à plaques tel que décrit dans le document WO2017097998, lequel décrit un distracteur à plaques, notamment un distracteur maxillo-facial à plaques, comportant par exemple : une première et une deuxième plaques de fixation, une tige filetée, un tube taraudé monté sur la tige filetée et fixé sur la deuxième plaque de fixation, une douille recevant d'une part la tige filetée libre en rotation et fixe en translation - également appelée vis sans fin - et d'autre part le tube taraudé fixe en rotation et libre en translation, un aimant permanent reçu dans un logement hermétiquement clos, s'étendant selon une direction principale définissant un axe principal et aimanté selon une direction transversale à la direction principale, monté dans le distracteur à plaques de telle sorte que la rotation de l'aimant sur lui-même, autour d'un axe de rotation parallèle à l'axe principal, provoque une rotation relative de la tige filetée par rapport au tube taraudé, l'aimant permanent étant situé à une distance d'une des extrémités du distracteur à plaques, mesurée selon la direction de l'axe de rotation de l'aimant, inférieure ou égale à 15 mm, de préférence inférieure ou égale à 10 mm, de manière plus préférée inférieure ou égale à 5 mm, par exemple à une extrémité dans le prolongement de la vis sans fin.

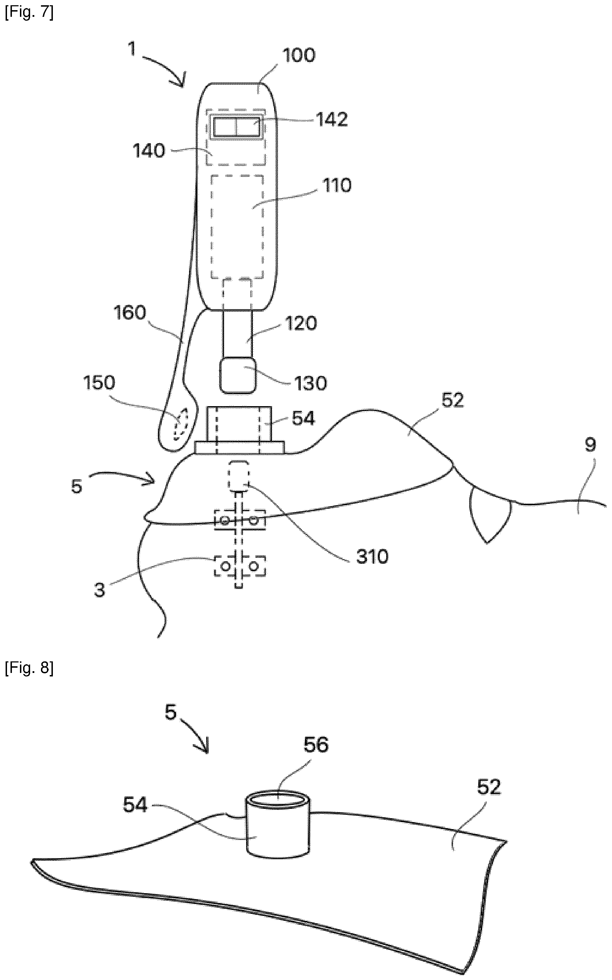

La figure 8 illustre un dispositif accessoire 5 d'aide au positionnement de l'outil d'activation 1 par rapport au distracteur à plaques 3.

La figure 1 représente un outil d'activation 1. L'outil d'activation 1 comprend un corps principal 100 à l'intérieur duquel sont logés une source d'énergie 105 (non représentée sur la figure 1) et un moteur électrique 110 prévu pour être alimenté par la source d'énergie 105.

La source d'énergie 105 est de préférence une batterie rechargeable au moyen d'une interface de charge sans-fil par induction. Le moteur électrique 110 est un moteur rotatif à courant continu comprenant deux bornes de connexion, un stator, un rotor, et un axe central de rotation. Le rotor du moteur électrique 110 est configuré pour opérer une rotation angulaire autour de l'axe de rotation du moteur 110. Le sens de rotation du rotor du moteur 110 dépend de la polarité de la tension électrique appliquée aux bornes de connexion du moteur 110. Le moteur électrique 110 est couplé à l'axe de rotation 120. Le couplage comprend éventuellement un ensemble mécanique de type réducteur, de sorte qu'une rotation d'un tour du rotor du moteur électrique 110 n'entraîne qu'une rotation d'une portion de tour de l'axe de rotation 120. L'utilisation d'un réducteur est d'autant plus avantageuse que la vitesse de rotation nominale du moteur est élevée.

Selon un mode de réalisation préféré de l'invention, l'axe de rotation 120 est mû par le moteur électrique 110 à basse vitesse de rotation, par exemple une vitesse entre 12 et 30 tours par minute, de préférence une vitesse de 18 tours par minute, pour chacun des deux sens de rotation. L'axe de rotation 120 se prolonge à l'extérieur du corps 100 de l'outil d'activation 1. L'axe de rotation 120 porte un élément magnétique 130 positionné à son extrémité située à l'extérieur du corps 100. Ainsi, l'élément magnétique 130 est configuré pour être entraîné par le moteur 110, via l'axe de rotation 120, à basse vitesse de rotation, par exemple au maximum entre 12 et 30 tours par minute, de préférence à 18 tours par minute. Cela évite de prévoir un réducteur dans le distracteur à plaques 3 entre l'élément magnétique 310 distant et la vis sans fin, et permet ainsi de limiter l'encombrement du distracteur à plaques 3. L'élément magnétique 130 est par exemple un aimant permanent, voire un aimant de type néodyme.

Avantageusement, l'utilisation d'un réducteur permet une plus grande précision dans le contrôle de la rotation de l'axe de rotation 120 et donc de la rotation de l'élément magnétique 130. Les termes « axe de rotation » doivent être interprétés ici comme un ensemble mécanique configuré pour transmettre la rotation angulaire du rotor du moteur 110 à l'élément magnétique 130. L'élément magnétique 130 est solidaire de l'axe de rotation 120. L'élément magnétique 130 peut être interchangeable ou non, en fonction de son mode de fixation sur l'axe de rotation 120.

Avantageusement, lorsque l'élément magnétique 130 est interchangeable, il est possible de l'adapter à une pluralité de types d'appareils d'expansion osseuse.

L'élément magnétique 130 est magnétisé de sorte que le champ magnétique qui en résulte soit orienté perpendiculairement ou sensiblement perpendiculairement à l'axe de rotation 120.

L'élément magnétique 130 est un aimant de type néodyme. L'homme du métier saura choisir un aimant néodyme en fonction des critères de distance entre l'élément magnétique 130 de l'outil d'activation 1 et l'élément magnétique 310 distant ainsi que du couple à transmettre sur la liaison mécanique d'expansion de l'appareil d'expansion à commander.

L'outil d'activation 1 comprend également un interrupteur 140 à 3 positions. Une première position du commutateur 140 à 3 positions correspond à une configuration entraînant la rotation du moteur 110 selon un premier sens de rotation (dit sens horaire). Une seconde position du commutateur 140 correspond à une configuration entraînant la rotation du moteur 110 selon un second sens de rotation, opposé au premier sens de rotation (dit sens antihoraire). Enfin une troisième position du commutateur 140 correspond à une absence de rotation du moteur.

L'outil d'activation 1 représenté sur la figure 1 est positionné en regard d'un distracteur à plaques 3 portant un élément magnétique 310 distant de l'élément magnétique 130 de l'outil d'activation 1. Le distracteur à plaques 3 est porté par un sujet nécessitant une expansion osseuse de type distraction osseuse. Le distracteur à plaques 3 est disposé dans le corps du sujet porteur.

L'élément magnétique 310 du distracteur à plaques est monté à l'extrémité d'une tige articulée apte à commander l'écartement de deux plaques. Un montage de l'élément magnétique 310 à l'extrémité de la vis sans fin du distracteur à plaques 3 autorise un couplage magnétique entre un élément magnétique externe au corps du sujet et l'élément magnétique 310 interne au corps du sujet. Les deux plaques du distracteur à plaques 3 sont configurées pour être fixées sur un os ou sur des os voisins du sujet nécessitant un allongement osseux. De tels mécanismes permettant de commander l'écartement de plaques à partir d'une vis sans fin sont connus. Il est également connu de disposer un élément magnétique à l'extrémité d'une vis sans fin d'un distracteur de sorte à pouvoir entraîner la vis sans fin en rotation à partir d'un élément magnétique extérieur au corps d'un sujet porteur d'un distracteur.

L'élément magnétique 310 du distracteur à plaques 3 est magnétisé de sorte que le champ magnétique qui en résulte soit perpendiculaire ou sensiblement perpendiculaire à l'axe de rotation qui le porte. Ainsi un couplage magnétique permettant un entrainement en rotation de l'élément magnétique 310 est possible avec un élément magnétique extérieur (tel que l'élément magnétique 130), positionné proche de l'élément magnétique 310, et magnétisé de sorte que le champ magnétique qui en résulte soit perpendiculaire ou sensiblement perpendiculaire à l'axe de rotation qui le porte.

Afin d'opérer un mouvement d'expansion osseuse, un utilisateur de l'outil d'activation 1 positionne celui-ci de sorte que l'élément magnétique 130 de l'outil d'activation soit positionné en regard de l'élément magnétique 310 du distracteur. Les termes « en regard » sont à interpréter ici comme signifiant que les éléments magnétiques rotatifs 130 et 310 sont positionnés de sorte que leurs axes de rotation coïncident sensiblement, et sont par exemple coaxiaux, de sorte à réaliser un couplage magnétique de nature à transmettre le mouvement de rotation angulaire de l'élément magnétique 130 à l'élément magnétique distant 310 du distracteur à plaques.

Après avoir positionné l'outil d'activation, l'utilisateur positionne le commutateur 140 de sorte à sélectionner le sens de rotation en correspondance d'un mouvement d'expansion osseuse, lorsque l'élément magnétique 310 sera mû en rotation sous l'effet du couplage magnétique avec l'élément magnétique 130 de l'outil d'activation 1.

Lorsque le commutateur 140 est positionné de sorte à sélectionner le sens de rotation en correspondance d'un mouvement d'expansion osseuse, la batterie rechargeable 105 délivre une tension adaptée aux bornes du moteur 101. Le rotor du moteur 101 opère alors une rotation transmise à l'axe de rotation 120 qui en est solidaire, ainsi qu'à l'élément magnétique 130. La rotation de l'élément magnétique 130 entraine la rotation de l'élément magnétique 310 distant du distracteur à plaques, positionné dans le corps du sujet, par couplage magnétique.

Le mécanisme interne du distracteur à plaques opère alors une expansion osseuse de type distraction.

La rotation dans le sens horaire de l'axe de rotation 120 de l'outil d'activation 1 entraîne par exemple un écartement des plaques du distracteur 3 lors d'un couplage magnétique entre l'outil est le distracteur. La rotation dans le sens antihoraire de l'axe 120 entraîne, à l'opposé, un rapprochement des plaques du distracteur 3.

L'utilisation d'un moteur électrique dont la fréquence (vitesse) de rotation est faible, ou l'utilisation d'un réducteur couplant l'axe de rotation du rotor au moteur 110 à l'axe de rotation 120 permet avantageusement de garantir un meilleur contrôle de la rotation de l'élément magnétique distant 310.

Avantageusement, la partie de l'axe de rotation 120 extérieure au corps 100 de l'outil d'activation 1 porte un ou plusieurs repères visuels permettant un dénombrement aisé et rapide du nombre de rotations de l'axe de rotation 120 et donc de l'élément magnétique 130.

Ces repères visuels peuvent être des marques, des parties colorées, ou tout autre élément visuel susceptible d'illustrer la réalisation d'un tour complet ou d'une portion de tour de l'axe de rotation 120 à l'utilisateur.

Selon un mode de réalisation préféré, l'axe de rotation 120 est de forme cylindrique. Selon des variantes, l'axe de rotation peut présenter une section transversale de forme carrée, pentagonale, hexagonale, octogonale, ou encore d'un nombre prédéterminé de pans latéraux dont une partie au moins porte des repères visuels aptes à illustrer aisément une rotation d'un nombre prédéterminé de portions de tour.

Dans le cas de la réalisation d'un mouvement entraînant un écartement des plaques du distracteur trop conséquent, l'utilisateur peut actionner le commutateur à 3 positions 140 de sorte à sélectionner une rotation inverse de celle entrainant l'écartement, effectuant ainsi une correction.

Lorsqu'une rotation de l'axe de rotation 120, et donc de l'élément magnétique 130 a été réalisée conformément au besoin, l'utilisateur positionne le commutateur à 3 positions dans la position en correspondance d'une absence de rotation du moteur.

Selon ce mode de réalisation, le commutateur à 3 positions opère comme un module de contrôle de la rotation de l'axe de rotation 120.

La **figure 2** est un schéma de principe de l'outil d'activation 1. Le commutateur 140 opérant comme un module de contrôle de la rotation de l'axe de rotation 120 est connecté entre la source de tension 105 et le moteur électrique 120.

La position des contacts du commutateur 140 permet une application d'une tension nominale de la source de tension 105 aux bornes du moteur électrique 110 à courant continu, ou l'application d'une tension inverse, ce qui correspond alors respectivement à une rotation du rotor du moteur selon un premier sens de rotation ou une rotation du rotor du moteur selon un second sens de rotation, opposé au premier sens de rotation.

Une position intermédiaire isole le moteur électrique 110 de la source d'alimentation 105, ce qui correspond alors à une absence totale d'alimentation du moteur se traduisant par une absence de rotation de son rotor et donc de l'élément de rotation 120. Le moteur électrique 110 est mécaniquement couplé à l'élément magnétique 130 par l'intermédiaire de l'axe de rotation 120.

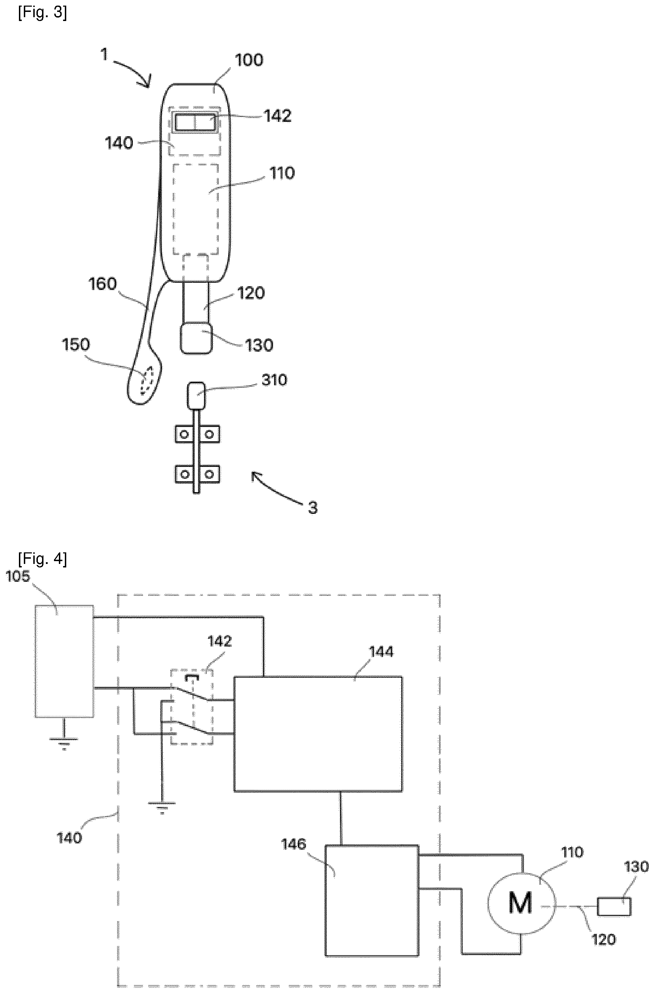

La **figure 3** représente l'outil d'activation selon un second mode de réalisation. Selon ce second mode de réalisation, le module 140 de contrôle de la rotation de l'axe de rotation 120 comprend une unité de contrôle embarquée configurée pour contrôler le moteur électrique 110 par le biais d'un circuit interface de puissance en fonction de la position d'un interrupteur à 3 positions 142.

Selon ce mode de réalisation, l'unité de contrôle embarquée du module de contrôle 140 de la rotation de l'axe comprend au moins un microcontrôleur adapté à contrôler le moteur électrique 110 à partir de signaux de contrôle fournis par le commutateur à 3 positions 142 et de signaux de contrôle représentatifs de la position et/ou de la rotation de l'axe de rotation 120. Les signaux de contrôles représentatifs de la position et/ou de la rotation de l'axe 120 sont générés à partir de moyens de détection de la rotation et de la position de l'axe 120, par exemple des moyens optoélectroniques, et appliqués en entrée de l'unité de contrôle.

Par exemple, l'axe de rotation 120 peut porter des ailettes latérales disposées de sorte à interrompre séquentiellement un signal lumineux ou infra-rouge émis et reçu par des moyens optoélectroniques couplés au microcontrôleur embarqué de l'unité de contrôle.

Les ailettes latérales permettant de détecter la rotation de l'axe de rotation 120 peuvent être implémentées sous la forme d'une roue, montée centrée sur l'axe de rotation 120 et comprenant des ouvertures régulièrement disposées en périphérie de la roue. Ce type de moyen de détection d'une rotation angulaire est bien connu et permet d'asservir le contrôle en rotation d'un élément, tel que l'axe de rotation 120, en fonction du mouvement réellement détecté. Avantageusement la capacité à détecter précisément la position de l'axe de rotation 120 et donc sa rotation permet de contrôler la vitesse de rotation par le biais de l'unité de contrôle.

La **figure 4** est un schéma de principe de l'outil d'activation selon le second mode de réalisation. Le commutateur à 3 positions 142 est connecté à des ports d'entrée du microcontrôleur de l'unité de contrôle 144. Le circuit interface de puissance 146 permet de contrôler la rotation du moteur électrique 110 à partir de ports de sortie du microcontrôleur de l'unité de contrôle 144.

L'interface de puissance 146 comprend notamment des circuits d'amplification et de protection aux fins de fournir des courants nécessaires à la rotation du moteur 110, à partir de ports de sortie élémentaires, telles que des sorties de type portes logiques, ou à collecteur ouvert de l'unité de contrôle.

L'unité de contrôle 144 comprend bien évidemment tous les éléments classiquement utilisés aux fins d'implémenter un contrôleur embarqué, tels que, à titre d'exemples non-limitatifs, un circuit de remise-à-zéro, un ou plusieurs circuits d'horloge, un module de mémoire volatile, utile à l'exécution de routines de logiciel embarqué, un module de mémoire non-volatile utile au stockage d'instructions de code exécutable pour l'exécution de routines logicielles, une circuiterie de mise en veille (mode basse consommation d'énergie), un circuit de supervision d'alimentation et/ou de contrôle de la charge de la batterie 105, un ou plusieurs convertisseurs analogique-numérique et numérique-analogique, des ports d'entrées et des ports de sorties, des entrées d'interruptions, des circuits de protections contre les décharges électrostatiques. Ces éléments n'étant pas détaillés plus encore, car n'étant pas utiles à la compréhension de l'invention.

Avantageusement, le logiciel embarqué, stocké dans une portion de mémoire non volatile, peut être mis à jour (actualisé) au travers d'une interface de communication prévue à cet effet. L'interface de communication peut être filaire ou sans fil. Le téléchargement d'une version actualisée de tout ou partie du logiciel embarqué peut se faire par couplage avec un terminal distant tel que, à titre d'exemple un ordinateur, une tablette ou un smartphone, connecté à internet ou disposant des éléments logiciels et matériels utiles à la mise à jour.

Selon un mode de réalisation de l'invention, l'unité de contrôle 144 est programmée pour générer des signaux de contrôle de la rotation du moteur électrique 110 en fonction de la position du commutateur à 3 positions 142 et de la rotation effective de l'axe de rotation 120.

Ainsi, si un utilisateur positionne le commutateur à 3 positions 142 dans la position définie pour opérer une rotation de l'axe de rotation 120 dans le sens prévu pour réaliser un écartement des plaques du distracteur 3 lorsque l'outil 1 est magnétiquement couplé au distracteur 3, l'unité de contrôle 144 commandera le circuit de puissance 146 pour contrôler la rotation du moteur dans le sens voulu jusqu'à ce que les moyens de détection et de position aient détecté une rotation angulaire correspondant à un nombre prédéterminé de tours ou de portions de tour de l'axe de rotation 120.

Lorsque l'axe 120 a été mû par le moteur électrique 110 selon un angle de rotation prédéterminé, l'unité de contrôle programmée pour mesurer l'angle de rotation à partir des signaux en provenance des moyens optoélectroniques inhibe les signaux de contrôle en rotation du moteur 110 de sorte que la rotation prenne fin.

Selon une variante du mode de réalisation, le moteur est un moteur pas-à-pas et le microcontrôleur de l'unité de contrôle 144 est configuré pour la génération de signaux adaptés au pilotage d'un moteur pas-à-pas au travers du circuit interface de puissance 146. Avantageusement, une telle variante permet de s'affranchir de l'utilisation de moyens de mesure de la position et/ou de la rotation angulaire de l'axe de rotation 120 puisque la rotation d'un moteur pas-à-pas est définie par une succession de mouvements de rotation élémentaires correspondant chacun à un pas et donc à un angle de rotation prédéfini autour de l'axe de rotation.

Selon une variante du mode de réalisation, l'unité de contrôle est programmée pour détecter un nombre d'impulsions (d'activation) sur le commutateur 142, sur une période prédéterminée, correspondant à la définition de nombre de tours ou de portions de tours à réaliser dans l'un des deux sens de rotation, de sorte à entraîner ensuite une rotation d'un nombre de portions de tour en correspondance du nombre d'impulsions détectées sur la période.

Ainsi, si par exemple un utilisateur active successivement dix fois le commutateur 142 pour générer un signal d'entrée sur l'unité de contrôle 144 de sorte à entrainer une rotation de l'axe 120 entrainant une expansion osseuse lors d'un couplage magnétique entre l'outil d'activation 1 et le distracteur 3, sur une période prédéterminée de 5 secondes, par exemple, l'unité de contrôle pilotera le moteur 110 de sorte à effectuer une rotation de l'axe 120 de dix pas angulaires élémentaires. Un pas angulaire élémentaire peut être, à titre d'exemple, un quart de tour, soit une rotation de 90 degrés de l'axe de rotation 120.

Selon une variante du mode de réalisation, le module de contrôle 140 comprend en outre une interface utilisateur adaptée à la saisie d'un nombre de tours ou de portions de tours à opérer, ainsi que d'une information représentative du sens de rotation désiré.

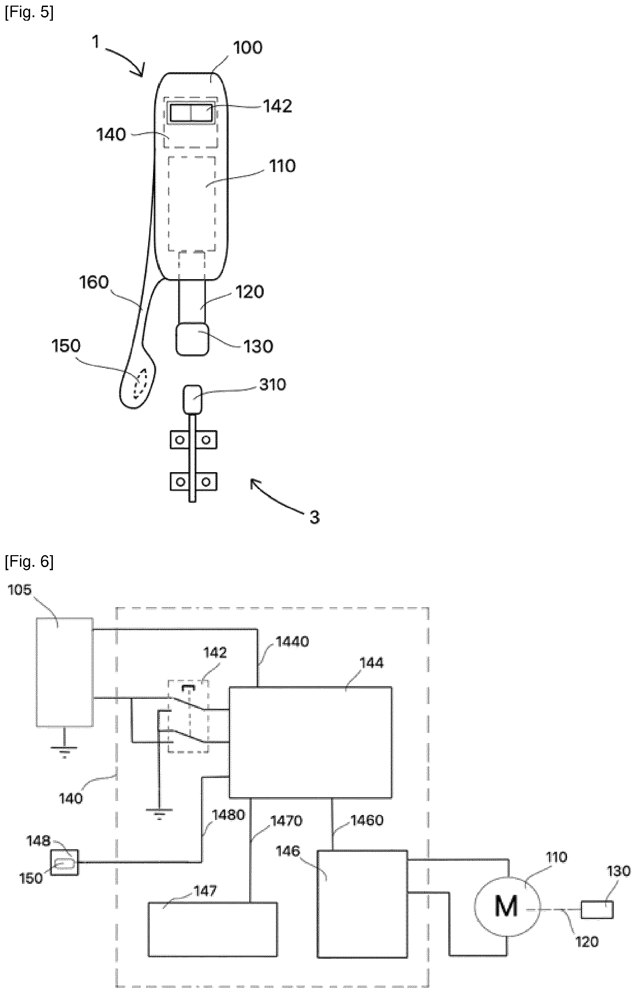

La **figure 5** illustre un outil d'activation 1 pour appareil d'expansion osseuse selon un troisième mode de réalisation. L'outil d'activation 1 comprend, selon ce troisième mode de réalisation, un bras 160 portant un capteur selfique 150 apte à mesurer des variations de champs magnétiques (un capteur de champ magnétique). Le bras 160 de l'outil d'activation 1, s'étendant à partir du corps 100 de l'outil d'activation 1, est configuré pour être positionné à proximité de l'élément magnétique 310 interne au sujet porteur d'un appareil d'expansion osseuse, lors d'un couplage magnétique entre l'élément magnétique 130 et un élément magnétique distant 310 d'un appareil d'expansion osseuse fixé dans le corps d'un sujet.

Le capteur selfique 150 est configuré pour détecter des variations de champ magnétique en utilisant l'effet Hall. Le capteur selfique 150 convertit les variations de champ magnétique en variations de tension.

Les variations de tension ainsi obtenues sont mesurées par l'unité de contrôle 144. A cet effet, l'unité de contrôle comprend un convertisseur analogique-numérique.

Le capteur selfique 150 constitue ainsi un capteur de champ magnétique, configuré pour mesurer une variation de champ magnétique émis par l'élément magnétique distant 310 porté par l'appareil d'expansion osseuse 3 lorsque l'élément magnétique distant 310 est positionné dans le prolongement de l'axe 120 de l'outil d'activation du côté de l'élément magnétique 130 solidaire de l'axe 120.

Avantageusement, l'utilisation du capteur de champ magnétique 150 permet une mesure précise d'un nombre de tours ou de portions de tours réellement effectués par l'élément magnétique 310 interne au sujet porteur du distracteur 3, et donc un meilleur contrôle de l'expansion osseuse.

La **figure 6** est un schéma de principe de l'outil d'activation 1 selon le troisième mode de réalisation pour lequel l'outil d'activation 1 comprend un capteur selfique 150 assemblé sur un support 148 proche de l'extrémité du bras 160 prolongeant le corps 100 de l'outil d'activation 1.

Selon ce troisième mode de réalisation, l'outil d'activation 1 comprend, outre l'unité de contrôle 144 alimentée par la connexion d'alimentation 1440, le circuit d'interface de puissance 146, reliée à l'unité de contrôle par une interface de connexion 1460 et l'interrupteur 142, un module interface utilisateur 147 connecté par le biais d'une interface de connexion 1470.

Avantageusement, le module interface utilisateur 147 comprend un module d'affichage adapté à la visualisation d'informations telles qu'un nombre de tours ou de portions de tours à opérer et un sens de rotation, ainsi que d'informations représentatives du nombre de tours ou de portions de tours à effectuer et/ou effectués.

Le module d'affichage comprend, par exemple un afficheur de type LCD (de l'anglais « Liquid Crystal Display » et qui signifie « afficheur à cristaux liquides » ou encore un affichage de type LED (de l'anglais « Light Emitting Device »), ces exemples étant non limitatifs.

Avantageusement le nombre de tours ou de portions de tours à opérer peut être saisi (programmé) par l'utilisateur de l'outil d'activation via une interface d'entrée connectée à l'unité de contrôle 144.

La saisie d'un nombre de tours ou de portions de tours peut s'effectuer au moyen d'un ensemble de touches ou en correspondance de fonctions prédéfinies ou encore permettant de se déplacer dans des menus contextuels affichés sur le module d'affichage. L'ensemble de ces fonctions étant réalisé sous contrôle de l'unité de contrôle 144, par l'exécution de routines logicielles embarquées prévues à cet effet et exécutées par le microcontrôleur de l'unité de contrôle 144.

Ainsi, un utilisateur de l'outil d'activation 1 peut le programmer pour réaliser un nombre de tours ou de portions de tours prédéfinis dans un sens de rotation donné. Par exemple, un utilisateur peut programmer l'outil d'activation 1 aux fins de contrôler une rotation dans le sens horaire correspondant à 3,75 tours.

Selon une variante, l'utilisateur peut incrémenter le nombre de tours ou de portions de tours à opérer par pressions successives sur une touche dédiée et une pression prolongée permet une remise à zéro du nombre de tours ou de portions de tours préprogrammé.

De façon similaire, le sens de rotation peut être défini, par exemple, par des pressions successives, temporellement rapprochées, qui permettent de dissocier la commande du sens de rotation de la définition du nombre de tours ou de portions de tours à effectuer.

D'autres types de codage de l'information à saisir peuvent bien évidemment être utilisés, le but étant pour l'utilisateur de pouvoir programmer un sens de rotation et un nombre de portions de tours à opérer avec l'outil d'activation 1.

Le module de contrôle 140 comprenant l'unité de contrôle 144 et ses périphériques 142, 146, 147, permettent ainsi de mieux contrôler le fonctionnement d'un appareil d'expansion osseuse tel que le distracteur à plaques 3, et de simplifier conséquemment les commandes d'expansion osseuse de distracteurs à plaques ou de disjoncteurs tout en apportant une meilleure précision.

Avantageusement, les moyens de contrôle (de saisie, de codage ou de programmation) d'un nombre de tours ou de portions de tours à réaliser dans un sens de rotation déterminé et les moyens d'affichage de l'outil d'activation 1 peuvent être combinés dans un même module ou élément, sous la forme d'un écran tactile, par exemple.

Ainsi, grâce à l'outil activation 1, il est possible pour un utilisateur non spécifiquement formé à ce type de manipulation, de commander une expansion osseuse, en réduisant considérablement les risques de procéder à une expansion osseuse insuffisante ou encore une expansion osseuse excessive.

Avantageusement, le contrôle de l'expansion osseuse peut ainsi être réalisé par un utilisateur non spécialiste ou non spécifiquement formé.

L'efficacité et la précision de l'outil d'activation sont d'autant plus importantes que le couplage magnétique entre les éléments magnétiques 130 de l'outil d'activation 310 et du distracteur 3 est performant.

Afin d'optimiser le couplage magnétique entre ces éléments, les éléments magnétiques 130 et 310 peuvent être remplacés par des éléments supports, porteurs d'une pluralité d'éléments magnétiques, magnétisés chacun de sorte à optimiser le couplage magnétique entre les deux éléments supports. Les éléments supports apparaissent alors par exemple comme des têtes cylindriques comprenant chacune des aimants cylindriques uniformément répartis autour d'un axe longitudinal. Ainsi, par exemple, l'élément magnétique 130 de l'outil d'activation 1 peut être remplacé par un support de tête cylindrique comprenant 3 éléments magnétiques distincts et l'élément magnétique 310 du distracteur 3 peut être remplacé par un support de tête cylindrique portant 3 autres éléments magnétiques distincts magnétisés de façon complémentaire à ceux de l'outil d'activation 1, afin de renforcer le couplage magnétique entre l'outil d'activation 1 et le distracteur 3. L'interaction coaxiale entre l'élément magnétique 130 et l'élément magnétique 310 est alors réalisée coaxialement à l'axe longitudinal.

Selon une variante des modes de réalisation décrits, et dans le cas où le couplage magnétique entre les éléments 130 et 310 est défini de façon optimale lorsque les éléments magnétiques 130 et 310 sont positionnés de façon coaxiale, c'est-à-dire lorsque l'axe de rotation de l'élément magnétique 130 et l'axe de rotation de l'élément magnétique 310 sont confondus, l'outil d'activation 1 peut être astucieusement positionné en regard du distracteur à plaques 3 à l'aide d'un dispositif accessoire 5 d'aide au positionnement de l'outil d'activation 1.

La **figure 7** illustre un positionnement de l'outil d'activation 1 en regard du distracteur à plaques 3 porté par un sujet 9. Le distracteur est fixé dans le corps du sujet 9, sur sa mandibule, du côté gauche de sa bouche. L'élément magnétique 310 est disposé dans le corps du sujet, non loin de sa bouche.

Avantageusement, le dispositif 5 d'aide au positionnement de l'outil d'activation 1 comprend une plaque en matériau thermo-formable 52 et un embout 54, sous la forme d'un embout creux. La cavité de l'embout 54 a une forme complémentaire de la forme de l'élément magnétique 130 de l'outil 1.

Le dispositif d'aide au positionnement 5 a été modelé par thermoformage pour se conformer à la physiologie du sujet 9 à l'occasion de la mise en place du distracteur à plaques 3, lors d'une intervention chirurgicale.

Lors de l'installation, le praticien a modelé la plaque 52 de matériau thermoformable pour définir un profil de surface complémentaire à celui du visage du sujet 9. Le modelage d'une telle plaque s'effectue simplement après élévation de température avec de l'eau chaude. L'embout 54 a alors été assemblé par sertissage, clipsage ou collage sur une ouverture de la plaque réalisée à l'endroit correspondant à la position de l'élément magnétique 310 disposé dans le corps du sujet 9 et invisible depuis l'extérieur après l'intervention.

L'embout 54 peut être assemblé sur la plaque 52 de matériau thermo-formable avant ou après modelage de celle-ci.

Ainsi, le dispositif d'aide au positionnement 5, modelé sur le visage du sujet 9, peut être repositionné simplement et rapidement sur le visage du sujet 9 ultérieurement à sa mise en forme, et définir ainsi, un guide de positionnement de l'élément magnétique 130, permettant d'être disposé rapidement et simplement dans l'alignement de l'élément magnétique 310 interne au sujet 9.

Lorsque le dispositif d'aide au positionnement 5 est positionné sur le visage du sujet 9, la cavité de l'embout 54 peut recevoir l'élément magnétique 130, alors disposé en regard de l'élément magnétique 310.

Avantageusement, cette configuration permet de surcroît de présenter le capteur selfique 150 proche de l'élément magnétique 310 interne au sujet et de mesurer alors la rotation effective de celui-ci par détection de la variation du champ magnétique reçue par le capteur selfique 150.

Selon un mode de réalisation de l'invention, la cavité de l'embout 54 comprend une butée afin d'éviter que l'élément magnétique 130 ne soit positionné au contact de l'épiderme du sujet 9 lors de la rotation de l'élément magnétique 130. Avantageusement, cela évite au sujet 9 de ressentir une gêne.

Selon un mode de réalisation de l'invention, l'embout 54 peut présenter une cavité sensiblement perpendiculaire à la surface de contact entre la plaque 52 et le visage du sujet 9, à l'endroit où est positionné l'embout 54. Selon des variantes, l'embout 54 présente une cavité dont l'axe longitudinal est orienté de façon oblique par rapport à la perpendiculaire à la surface de contact entre la plaque 52 et le visage du sujet 9.

Cela permet avantageusement de positionner l'élément magnétique 130 de façon coaxiale avec l'élément magnétique 310, en fonction de la position du distracteur à plaques 3 dans le corps du sujet 9.

La **figure 8** représente le dispositif d'aide au positionnement 5 de l'outil d'activation 1, avant mise en forme de la plaque de matériau thermo-formable 52 sur un sujet. La plaque de matériau thermo-formable 52 porte un embout 54 présentant une ouverture cylindrique 56 configurée pour recevoir l'élément magnétique 130 de l'outil d'activation 1.

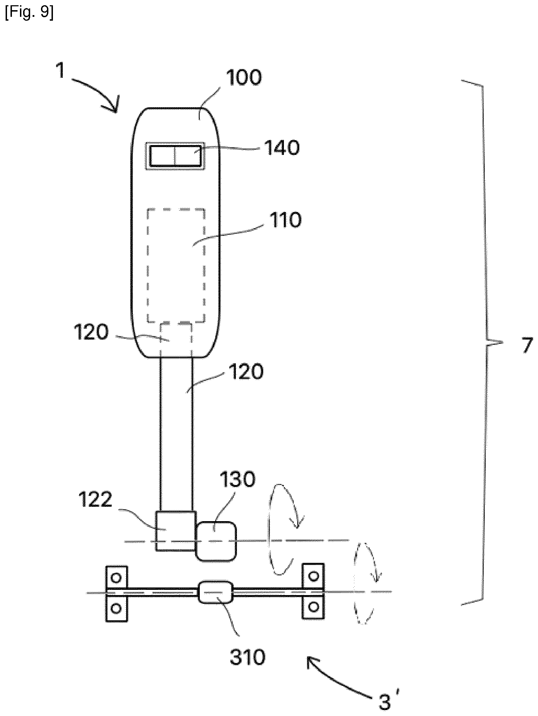

La **figure 9** représente une variante de l'outil d'activation selon un mode de réalisation non limitatif pour laquelle l'axe de rotation 120 comprend une partie angulaire 122 à engrenages internes permettant d'entrainer l'élément magnétique 130 autour d'un axe perpendiculaire à celui de la rotation du moteur 110. La partie angulaire 122 comprend typiquement un renvoi angulaire conique comprenant deux pignons coniques.

Sur la figure 9, l'outil d'activation 1 est représenté positionné en regard d'un appareil d'expansion 3' de type disjoncteur, prévu pour disjoindre des parties osseuses d'un sujet. Par exemple, l'appareil 3' peut être fixé solidairement de parties latérales droite et gauche de la gencive supérieure d'un sujet, en vue de réaliser un agrandissement progressif du palais. Le disjoncteur 3' peut être configuré pour être fixé sur des parties osseuses, sur des bagues rendues solidaires de dents d'un sujet, ou encore à des arcs dentaires (cas de distraction maxillaire ou symphysaire), par exemple.

L'élément magnétique 310 du disjoncteur est solidaire d'un axe de type tige filetée comprenant des liaisons à vis sans fin avec les éléments de fixation situés aux extrémités de la tige. Les deux filetages présentent des pas inversés.

Les filetages de part et d'autre de l'élément magnétique 310 sont ainsi tels que lorsqu'une rotation de l'élément magnétique 310 est réalisée dans un sens, les éléments de fixation s'écartent pour disjoindre les parties qui y sont reliées. Inversement, lors d'une rotation de l'élément magnétique 310 est effectuée dans le sens opposé, les éléments de fixations se rapprochent.

En d'autres termes, l'outil d'activation 1 permet de contrôler l'expansion osseuse réalisée par un appareil d'expansion osseuse, tel que le distracteur à plaques 3 ou le disjoncteur 3'. L'outil d'activation 1 comprend l'élément magnétique 130 solidaire de l'axe de rotation 120 de l'outil d'activation 1. L'élément magnétique 130 est configuré pour interagir avec l'élément magnétique distant 310 porté par l'appareil d'expansion osseuse 3. L'outil d'activation 1 comprend en outre le moteur électrique 110 configuré pour opérer la rotation de l'axe 120, et le module de contrôle 140 de la rotation de l'axe 120.

L'outil d'activation 1 est configuré pour contrôler le sens de rotation de l'axe 120.

Le module de contrôle 140 est configuré pour contrôler la vitesse de rotation de l'axe 120.

L'outil d'activation comprend des moyens de comptage d'un nombre de tours ou de portions de tours réalisés lors de la rotation de l'axe 120.

Le module de contrôle 140 de l'outil d'activation 1 comprend des moyens d'arrêt de la rotation de l'axe lorsque l'axe a opéré une rotation d'un nombre prédéterminé de tours.

Le module de contrôle 140 comprend, via le microcontrôleur interne à l'unité de contrôle 144 des moyens de programmation d'un sens de rotation et d'un nombre de tours de l'axe à opérer selon ce sens de direction

L'outil d'activation 1 comprend le capteur selfique 150 de champ magnétique 150 pour mesurer une variation de champ magnétique émis par l'élément magnétique distant 310, porté par l'appareil d'expansion osseuse 3 lorsque l'élément magnétique distant 310 est positionné dans le prolongement de l'axe 120 de l'outil d'activation 1, du côté de l'élément magnétique 130 solidaire de l'axe 120.

L'outil d'activation 1 comprend un module de mesure de champ magnétique pour déterminer une rotation de l'élément magnétique distant 310 d'au moins un nombre de tours ou de portions de tours prédéterminé autour de l'axe d'écartement de l'appareil d'expansion osseuse 3. Le module de mesure est par exemple le capteur de champ magnétique 150.

L'appareil d'expansion osseuse 3 et l'outil d'activation 1 composent un ensemble 7 utile à la réalisation d'opération d'expansion osseuse permettant une plus grande précision et simplifiant la réalisation d'opérations successives d'expansion osseuse par un utilisateur non spécifiquement formé.

L'ensemble 7 utile à la réalisation d'opérations d'expansion osseuse peut être avantageusement complété par le dispositif 5 d'aide au positionnement de l'élément magnétique de l'outil 1, et donc de l'élément magnétique 130 par rapport à l'élément magnétique 310 porté par l'appareil d'expansion osseuse 3.

L'ensemble 7 comprend alors le dispositif 5 d'aide au positionnement de l'élément magnétique 130 de l'outil 1, lequel comprend une feuille en matériau thermoformable 52 pouvant pour être rapportée et formée sur le sujet 9 et comportant le guide de positionnement 54 de l'élément magnétique 130.

En outre, selon des variantes, l'axe de rotation 120 peut être remplacé par un système rotatif non rectiligne comprenant des éléments mécaniques intermédiaires, tels que des engrenages ou des cardans configurés pour transmettre la rotation angulaire du moteur 110 à l'élément magnétique 130. Ainsi une rotation de l'élément magnétique 130 peut être réalisée autour d'un axe différent de l'axe principal du corps 100 de l'outil d'activation 1 et s'adapter à des dispositifs distracteurs ou disjoncteurs de formes variées.

Selon ces variantes, il est avantageusement possible de s'adapter à différentes configurations de dispositifs d'expansion osseuse et en particulier à différentes configurations de positionnement de l'axe de rotation de l'élément magnétique distant 310.

Selon une autre variante, le système rotatif non rectiligne permettant de transmettre la rotation angulaire du moteur 110 à l'élément magnétique 130 comprend un câble souple porté à l'intérieur d'une gaine, la gaine étant elle-même portée par un tube semi-rigide dont la forme peut être modifiée manuellement aux fins de s'adapter à différents types d'appareils d'expansion osseuse.

Selon une autre variante, l'outil d'activation 1 comprend une interface de communication sans-fil adaptée aux communications bidirectionnelles de courtes distances. L'interface sans-fil peut être de type compatible avec la norme de communication sans fil communément appelée Bluetooth ^{®} ou l'une quelconque de ses évolutions.

Selon une autre variante, l'interface de communication sans-fil peut être de type WiFi ^{®}, ou l'une quelconque de ses évolutions.

Avantageusement, l'utilisation d'une interface de communication sans-fil permet d'utiliser une unité de contrôle distante, telle que, par exemple, l'unité de contrôle d'un dispositif smartphone, sous contrôle d'une application logicielle dédiée.

Avantageusement, l'utilisation d'une telle interface permet un transfert à un tiers distant, d'informations utiles au traitement d'un sujet, tel que, par exemple, des données représentatives de mesures opérées par le capteur magnétique 150 de l'outil d'activation 1. Ce tiers peut être par exemple un proche d'un sujet à qui l'on opère une distraction, ou encore un praticien tel que son médecin, alors en capacité de contrôler à distance le bon déroulement des opérations de distraction osseuse.

L'utilisation d'une unité de contrôle distante permet d'utiliser un logiciel embarqué relativement sommaire dans l'outil d'activation 1 et de faciliter des évolutions ou des mises à jour des moyens de commande alors principalement implémentés sur le smartphone.

Un autre avantage est de permettre une configuration à distance de l'équipement, par exemple par un praticien.

En outre, une utilisation des moyens d'un smartphone permet de simplifier l'architecture des systèmes embarqués de l'outil d'activation 1.

Selon une variante, la source d'énergie 105 est une batterie amovible, non rechargeable. Selon une autre variante, la source d'énergie 105 est un module d'alimentation à découpage prévu pour être raccordée au réseau électrique dit « secteur » par l'intermédiaire d'une prise murale.

Selon une variante, l'axe 120 est un axe fixe et l'outil d'activation ne comprend pas de moteur. Selon cette variante, l'élément magnétique 130 se compose d'une pluralité de bobines électromagnétiques disposées de façon à former un élément magnétique de forme circulaire ou sensiblement circulaire, périphérique à l'axe 120, contrôlées en alimentation par le module de contrôle 140, et configurées pour permettre la génération d'un champ magnétique tournant orienté perpendiculairement à l'axe 120. Une telle architecture est alors apte à la génération d'un champ magnétique similaire à celui créé lorsque l'élément magnétique 130 est un aimant.

L'invention ne se limite pas aux seuls modes de réalisation décrits ci-avant et des caractéristiques décrites dans chacun des modes de réalisations précités peuvent être combinées, pour autant qu'elles relèvent du champ d'application des revendications. L'ensemble de l'invention revendiquée comporte également un dispositif d'aide au positionnement d'un équipement par rapport à un support dans un objet ou dans le corps d'une personne.

On connaît, dans l'état la technique, notamment d'après la demande de brevet WO2017097998, un distracteur à plaques implanté dans le corps d'un sujet et prévu pour opérer une distraction maxillo-faciale.

Un tel distracteur comprend deux plaques fixées sur un côté d'un os préalablement fracturé ou sur le côté d'os voisins du sujet porteur et comprend un aimant susceptible d'être mû en rotation à proximité d'une extrémité du distracteur.

Le distracteur peut également être utilisé à des dents ou à des bagues d'un appareil dentaire.

Dans un tel distracteur, l'aimant est cylindrique et magnétisé selon une direction perpendiculaire à son axe longitudinal. L'aimant est en outre solidaire d'une vis sans fin dont la rotation entraîne la translation d'un tube taraudé, ce qui permet l'allongement de la distance entre deux plaques.

L'aimant est donc positionné à l'intérieur du corps, et le distracteur peut être actionné depuis l'extérieur par couplage magnétique ou non magnétique (mécanique).

Il est ainsi possible de commander l'allongement du distracteur à plaques depuis l'extérieur du patient, sans contact et sans activateur traversant la peau ou une muqueuse du patient. Grâce à ce système, il n'est pas nécessaire de mettre en œuvre une tige articulée traversant les tissus du patient.

Il suffit de positionner un aimant par exemple cylindrique magnétisé en vis-à-vis de l'aimant interne pour que la rotation de l'aimant externe autour de son axe provoque la rotation de l'aimant interne.

Dans le cas d'un couplage magnétique, le couplage est efficace si les aimants sont correctement positionnés l'un par rapport à l'autre et que leurs axes respectifs coïncident ou se rapprochent le plus possible l'un de l'autre.

Un inconvénient de système est qu'il est difficile de positionner un équipement extérieur (par exemple un outil d'activation) par rapport à un élément (une cible) intérieur au corps, et donc invisible ou très peu visible depuis l'extérieur.

L'invention a notamment pour but de faciliter le positionnement d'un outil d'activation par rapport à un distracteur à plaques.

A cet effet l'ensemble conforme à l'invention comporte un dispositif d'aide au positionnement d'un outil d'activation par rapport à un distracteur à plaques, comprenant une feuille en matériau formable ou thermo-formable configurée pour être rapportée sur un sujet et comportant un guide de positionnement de l'élément magnétique de l'outils d'activation.

Ainsi, on propose d'utiliser une feuille que l'on conforme à la surface du sujet et comportant en outre un guide de positionnement. Il en résulte que, non seulement l'équipement est guidé, donc bien positionné par rapport à la feuille, mais en outre, la feuille peut prendre exactement la forme du support, ce qui évite des changements de position de la feuille par rapport au sujet.

Ce dispositif d'aide au positionnement est particulièrement intéressant pour le positionnement d'un équipement extérieur au corps d'un sujet par rapport à la position d'un objet cible intérieur au corps du sujet, et donc par rapport à une partie de la surface du corps du sujet. Il est ainsi possible, de façon particulièrement avantageuse, de faciliter le positionnent d'un outil d'activation d'un distracteur à plaques, externe au corps d'un sujet, par rapport au distracteur à plaques, interne au corps du sujet, et ainsi faciliter le contrôle de ce distracteur.

Le terme « formable » est à interpréter ici de préférence comme un matériau rigide présentant une première forme et susceptible d'être conformé ou modelé par des opérations simples de mise en forme afin d'obtenir une seconde forme qui sera conservée, sauf à réitérer des opérations de mise en forme. Un tel matériau est par exemple du plâtre ou une résine durcissante au contact de l'air.

Le terme « thermo-formable » est à interpréter de préférence comme un matériau formable au sens défini ci-avant et pour lequel des opérations de mise en forme requièrent une modification de la température du matériau, par exemple une exposition à la chaleur ou un trempage dans un bain à une température prédéfinie.

Suivant d'autres caractéristiques optionnelles du dispositif d'aide au positionnement, prises seules ou en combinaison :
- Le guide de positionnement délimite une cavité de forme prédéterminée configurée pour le positionnement d'une partie de l'équipement à positionner présentant une forme complémentaire à celle de la cavité. Le positionnement d'un équipement dont la forme, au moins partielle, est complémentaire à celle de la cavité est ainsi optimisé et efficace. Le positionnement d'un équipement par rapport au guide peut ainsi être prédéfini dans tous les axes de positionnement, notamment par l'utilisation d'une forme formant un détrompeur.
- La cavité du guide de positionnement est traversante. Il est ainsi possible d'approcher l'élément magnétique de l'outil d'activation au plus près de l'élément magnétique du distracteur à plaques.
- La cavité du guide est de forme sensiblement cylindrique ou sensiblement tronconique. Cela permet de faire coïncider les axes longitudinaux de l'équipement (externe) et d'un équipement interne à un sujet sur lequel est disposé le dispositif d'aide au positionnement ou encore de reproduire le bon positionnement d'un équipement externe par rapport à une cible interne, c'est-à-dire de répéter l'angulation prévue entre des axes repérés des deux équipements. L'utilisation d'une cavité cylindrique permet l'insertion d'un aimant cylindrique néodyme magnétisé perpendiculairement à son axe longitudinal et son alignement coaxial avec l'axe d'un aimant similaire d'un équipement interne à un sujet et avec lequel il convient de réaliser un couplage magnétique. Dans le cas où la forme est tronconique, cela permet en outre de définir une distance maximale avec la cible, en fonction de l'équipement utilisé.
- Le guide de positionnement est fixé sur la feuille de matériau formable ou thermo-formable par clipsage, sertissage, soudure ou collage. L'assemblage du dispositif d'aide au positionnement est ainsi rendu aisé et rapide. Il peut se faire au cours d'une intervention visant à implanter un équipement dans le corps d'un sujet ou en marge de celle-ci.
- La cavité présentant un axe longitudinal formant un angle de 1 à 90 degrés avec un plan comprenant une base du guide en contact avec la feuille de matériau formable ou thermo-formable. Il est ainsi possible de prédéfinir un positionnement d'un équipement selon une orientation spatiale prédéfinie lorsqu'un équipement interne au sujet le requiert pour son utilisation ou son activation. Par exemple, un aimant néodyme monté en rotation dans un équipement implanté dans le corps d'un sujet peut présenter une orientation de son axe de rotation non perpendiculaire avec la surface du corps du sujet à l'intersection entre l'axe de rotation et la surface du corps.
- Le matériau formable ou thermo-formable est choisi dans un groupe comprenant une plaque thermo-formable, du plâtre, du papier mâché, de l'argile, de la résine ou un matériau utilisable pour une impression 3D. Il est alors possible de le modeler simplement, en le transformant par l'utilisation de la chaleur, ou avec de l'eau, selon le matériau et sans avoir à effectuer d'opérations plus complexes.
- Le guide de positionnement de l'équipement est réalisé dans un matériau choisi dans le groupe comprenant du PEEK (polyétheréthercétone), du polyuréthane, du titane, de l'acier inoxydable, de l'or, du plâtre, de la résine, ou du verre. Avantageusement ces matériaux sont compatibles avec un contact avec l'épiderme d'un sujet.
- Le dispositif est prévu pour le positionnement d'un équipement de type outil d'activation pour un distracteur à plaques sur un sujet. Il est ainsi possible d'optimiser le couplage magnétique entre un aimant rotatif d'un distracteur à plaques et un aimant rotatif d'un activateur pour un tel distracteur, de sorte à optimiser le couplage magnétique entre ces deux équipements. En outre, cela confère une meilleure capacité de contrôle d'un distracteur interne à un sujet et sans requérir d'accès transcutané.
- La feuille en matériau formable ou thermo-formable présente au moins partiellement la forme d'un visage. Il est ainsi avantageusement possible de conformer la feuille en matériau formable ou thermo-formable sur des parties d'un visage susceptible de recouvrir un équipement interne, tel que, à titre d'exemple, un distracteur pour une application maxillo-faciale.

Une méthode de fabrication d'un dispositif d'aide au positionnement d'un équipement comprend les étapes de :
- élévation en température d'une feuille en matériau formable ou thermo-formable,
- mise en forme de la feuille de matériau formable ou thermo-formable élevée en température par moulage sur un objet,
- perforation de la pièce de matériau formable ou thermo-formable de façon à former une ouverture, et
- fixation d'un guide de positionnement de l'équipement dans ou en regard de l'ouverture, avant ou après les étapes d'élévation en température et de mise en forme de la feuille de matériau.

Une telle méthode permet de fabriquer rapidement, par exemple au cours d'une intervention chirurgicale visant une implantation d'un équipement dans un sujet, ou en marge de celle-ci, un dispositif d'aide au positionnement d'un équipement externe. Par exemple, il est possible de fabriquer un guide d'aide au positionnement d'un activateur de distracteurs à plaques, lors de l'intervention visant à implanter le distracteur à plaques. Avantageusement, et grâce à la mise en oeuvre de la méthode ci-dessus, un praticien (chirurgien ou infirmier, par exemple) peut fabriquer un dispositif d'aide au positionnement d'un équipement ou outil en correspondance des caractéristiques d'implantation, d'orientation ou d'utilisation d'un équipement implanté dans le corps d'un sujet.

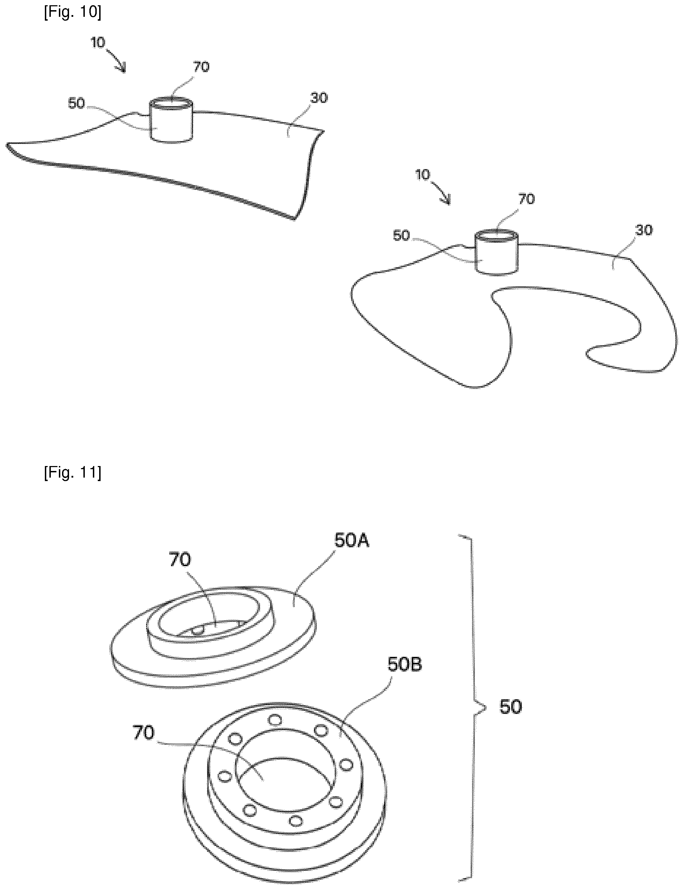

On a représenté sur les figures 10 à 16 un dispositif 10 d'aide au positionnement d'un équipement.

La **figure 10** représente le dispositif 10 d'aide au positionnement d'un équipement sur un support. Le dispositif 10 comprend une feuille en matériau formable ou thermo-formable 30 et un guide 50 de positionnement pour faciliter le positionnement d'un équipement.

Le guide 50 présente une cavité 70, ici de forme cylindrique. La cavité 70 peut comprendre une ouverture de sorte qu'elle soit débouchante sur un support (objet ou partie de corps) sur lequel le dispositif est appliqué.

Sur la partie en haut à gauche de la figure 10, la feuille de matériau formable ou thermo-formable 30 n'a pas été préalablement formée pour s'adapter à un support. Sur la partie en bas à droite de la figure 10, on peut voir le dispositif 10 après que la feuille en matériau formable ou thermo-formable 30 ait été formée pour se conformer à un support. Par exemple, la feuille 30 a été formée sur une partie inférieure du visage d'un sujet, tel que le bas d'une joue, situé proche du menton, et en regard d'une partie de la mandibule d'un sujet.

La feuille 30 en matériau formable ou thermo-formable est composée de résine thermo-formable dont une mise en forme peut être opérée suite à une élévation de température (bain d'eau chaude, par exemple). Selon des variantes la feuille 30 est composée d'un matériau formable mais non thermo-formable susceptible d'être modelé et de conserver sa forme après modelage. Par exemple, la feuille de matériau 30 peut être une bandelette de plâtre ou un assemblage de bandelettes de plâtre. Selon d'autres variantes, la feuille de matériau 30 peut être réalisée en argile ou dans un matériau classiquement utilisé pour les impressions en trois dimensions (par une imprimante 3D). Dans ce cas, il est important que le matériau soit conforme aux normes imposées par une utilisation visant un contact de la feuille de matériau avec la peau. Ces exemples de matériaux ne sont pas limitatifs.

Le guide 50 est réalisé dans un matériau de préférence rigide, tel que, à titre d'exemple du PEEK, du polyuréthane, du titane, de l'acier inoxydable, de l'or, du plâtre, de la résine, du verre, ou encore un matériau classiquement utilisé pour les impressions en trois dimensions conforme aux normes imposées par une utilisation visant un contact de la feuille de matériau avec la peau, cette liste étant non exhaustive.

La feuille de matériau 30, encore appelée « partie surfacique » du dispositif d'aide au positionnement 10 peut, selon une variante, ne pas présenter d'ouverture, en vis-à-vis du guide 50 (c'est-à-dire à l'endroit de positionnement du guide 50 sur la feuille 30). Le guide 50 est également communément appelé « partie volumique ».

En offrant la possibilité de conformer la feuille de matériau 30 à un support quelconque, le dispositif permet avantageusement d'adapter avec une grande précision des opérations à la morphologie d'un sujet ou à la topologie d'une surface frontière délimitant l'intérieur et l'extérieur d'un objet.

Les opérations ainsi rendues possibles ou plus aisées sont par exemple des actes médicaux ou paramédicaux apportés à une personne et qui se réfèrent à un élément cible interne au corps de cette personne.

L'élément auquel se réfère une opération peut être précédemment introduit dans le corps de la personne concernée ou encore faire biologiquement ou structurellement partie du corps de la personne. Conformément à l'invention, l'élément interne auquel un acte doit se référer est un distracteur à plaques utilisé en vue d'une expansion osseuse

Avantageusement et selon l'utilisation prévue, la cavité 70 du guide 50 peut présenter des formes variées.

Par exemple, la cavité 70 peut présenter une section transversale d'alésage circulaire, triangulaire, sous forme de quadrilatère ou encore sous forme d'un polygone dont le nombre de côtés est supérieur à 4.

Une section transversale d'usinage de la cavité 70 du guide 50 peut correspondre à des triangles remarquables, à un carré, un rectangle, un losange.

La cavité 7 peut avoir un volume cylindrique, sous forme de cône, parallélépipédique ou encore sous une forme quelconque, complémentaire de la forme de tout ou partie d'un outil ou d'une sonde.

Différentes ouvertures peuvent être prévues entre l'intérieur et l'extérieur de la cavité 70 du guide 50, notamment pour évacuer un excès de produit lubrifiant utilisé pour faciliter l'introduction d'un outil ou d'un équipement dans la cavité 70. De telles ouvertures sont alors préférentiellement orientées selon un axe perpendiculaire à l'axe longitudinal de la cavité 70.

Selon un mode de réalisation, le guide 50 est composé de deux pièces 50A et 50B respectivement appelées demi-guide supérieur et demi-guide inférieur.

La **figure 11** est une vue éclatée d'un guide 50 pour le dispositif 10 avant assemblage. Le demi-guide supérieur 50A comprend des pions en saillie configurés pour un couplage en force, par emboîtement, dans des cavités complémentaires du demi-guide inférieur 50B. Le demi-guide inférieur 50B présente une embase cylindrique et un tronc creux ouvert plus étroit, également cylindrique, formant une cavité, et s'étendant selon un axe longitudinal à partir de l'embase.

Lorsque le demi-guide supérieur 50A, qui présente également une embase, est emboité en force dans le demi-guide inférieur 50B, les deux embases sont configurées pour enserrer la feuille de matériau formable ou thermo-formable 30 entre leurs surfaces respectives.

Le guide représenté **figure 11** est tel que l'axe longitudinal de la cavité est perpendiculaire à la surface des embases, et donc à la surface de la feuille de matériau 30, à l'endroit d'insertion du guide 50.

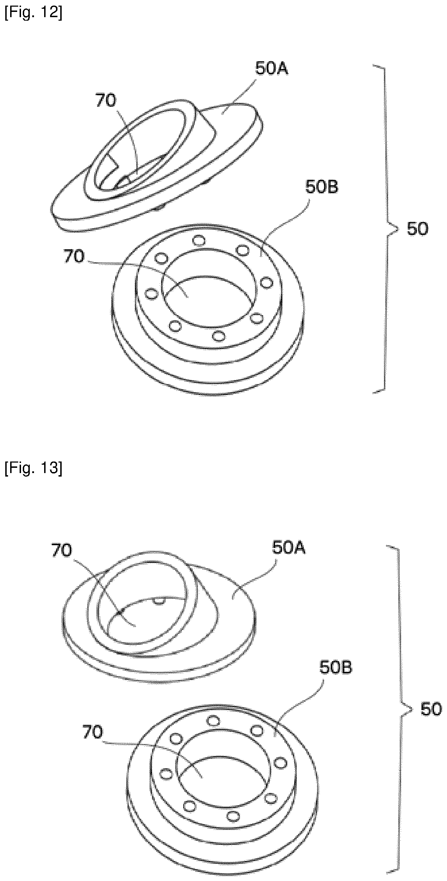

Selon une première variante, représentée sur la **figure 12****,** le demi-guide supérieur 50A est configuré de sorte que l'axe longitudinal de la cavité du guide soit incliné selon un angle de 15 degrés par rapport au plan de son embase.

La cavité ainsi orientée permet de guider un positionnement d'un équipement selon un angle d'inclinaison de 15 degrés par rapport à la surface de la feuille de matériau 30, à l'endroit du guide 50.

Selon une seconde variante, représentée sur la **figure 13****,** le demi-guide supérieur 50A est configuré de sorte que l'axe longitudinal de la cavité du guide soit incliné selon un angle de 30 degrés par rapport au plan de son embase.

La cavité ainsi orientée permet de guider un positionnement d'un équipement selon un angle d'inclinaison de 30 degrés par rapport à la surface de la feuille de matériau 30, à l'endroit du guide 50.

Selon une variante du mode de réalisation, l'assemblage du demi-guide supérieur 50A sur le demi-guide inférieur 50B peut être réalisé par collage ou soudure de sorte à former un ensemble rigide comprenant les demi-guides 50A et 50B et la feuille de matériau 30 autours des embases.

Selon une autre variante, et dans le cas d'un assemblage par collage ou soudure, le guide 50 peut comprendre uniquement le demi-guide supérieur 50A et il est possible de s'affranchir de l'utilisation du demi-guide inférieur 50B. Dans ce cas, les pions du demi-guide 50A peuvent ne pas exister.

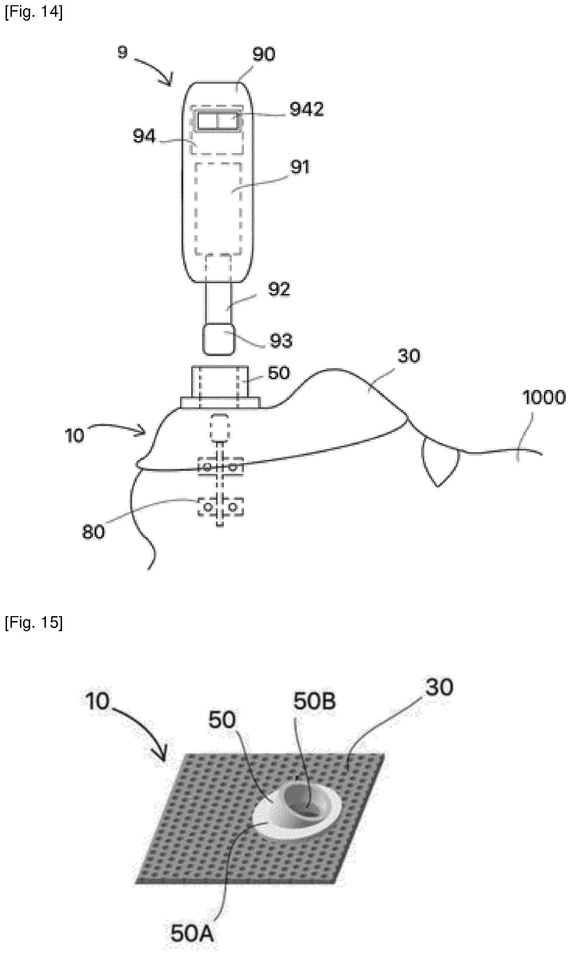

**La** **figure 14** illustre une utilisation d'un dispositif 10, pour le positionnement d'un outil d'activation 9 d'un distracteur à plaques 80 positionné sur la mandibule d'un sujet 1000 en vue d'une expansion osseuse maxillo-faciale. Le distracteur à plaques 80 comprend un axe porteur en son extrémité d'un aimant permanent rotatif magnétisé perpendiculairement à son axe de rotation et dont la rotation entraine un écartement des plaques pour opérer une expansion osseuse, par utilisation d'un système à vis sans fin.

La feuille 30 du dispositif 10 a été modelée sur le visage précédemment à l'opération de distraction osseuse à réaliser, tel que, par exemple au cours de l'intervention chirurgicale visant à implanter le distracteur à plaques 80 dans le corps du sujet 1000, ou peu après celle-ci par une personne ayant connaissance des caractéristiques d'implantation du distracteur (dimensions, orientation).

Lors de la mise en forme et de l'assemblage du dispositif 10, la feuille 30 a été perforée pour réaliser une ouverture et le guide 50 a été assemblé et/ou positionnée sur cette ouverture, de sorte que, la cavité du guide 50 permette un guidage précis et à une distance prédéfinie d'un élément magnétique utile à l'activation par couplage magnétique du distracteur à plaques 80 à travers les tissus du sujet 1000.

L'outil d'activation 9 représenté sur la **figure 14** est configuré pour activer le distracteur à plaques 80 par entrainement en rotation de l'aimant interne du distracteur à plaques. A cet effet l'outil d'activation 9 présente un corps 90 pour sa préhension. Le corps 90 de l'outil 9 comprend un moteur à courant continu 91 et un axe de rotation 92 solidaire de l'axe de rotation du moteur 91, portant en son extrémité un aimant permanent 93 magnétisé perpendiculairement à son axe de rotation. L'outil 9 comprend un module de contrôle 94 du sens et de la vitesse de rotation du moteur 91 à partir d'un interrupteur à 3 positions 942. Les positions de l'interrupteur 942 correspondant respectivement à une rotation du moteur dans un premier sens, un arrêt du moteur et une rotation du moteur dans un second sens, opposé au premier sens.

L'utilisation du dispositif 10 d'aide au positionnement d'un équipement permet avantageusement de faire coïncider les axes de rotation de l'aimant de l'outil 9 et de l'aimant du distracteur à plaques 80, ainsi que de définir une distance optimale entre les deux aimants pour optimiser le couplage magnétique et donc le contrôle du distracteur à plaques 80.

Avantageusement, la cavité 70 du guide 50 configurée pour l'introduction de l'aimant 93 de l'outil 9 peut comprendre une butée, par exemple sous la forme d'une collerette intérieure à la cavité du guide 50, de sorte à éviter un frottement entre l'aimant 93 et l'épiderme du sujet 1000. La même fonction de butée peut être réalisée par la feuille 30 de matériau, en l'absence d'ouverture dans celle-ci en regard de la cavité.

La **figure 15** illustre un assemblage d'un guide 50 orienté selon une inclinaison de 15 degrés par rapport à une perpendiculaire d'une feuille 30 à l'endroit recevant le guide 50 pour former un dispositif 10, avant mise en forme de le feuille 30 sur un support tel que, par exemple, la mandibule d'une personne.

Le dispositif 10 peut être fourni prêt à l'emploi dans des versions variées comprenant une grande variété de formes de guide 50. Ainsi, il est possible pour un praticien, tel que par exemple un chirurgien ou un infirmer, de préparer le dispositif d'aide au positionnement en sélectionnant un modèle dans une gamme approprié à l'usage et en modelant la feuille de matériau formable ou thermo-formable du modèle sélectionné sur le corps d'un sujet. Le dispositif 10 alors préparé sera adapté à des actes médicaux ou paramédicaux à venir. Ces actes s'en verront simplifiés, en particulier pour une personne peu habituée ou non spécifiquement formée pour les réaliser (actes d'imagerie médicale, d'injection, d'activation, par exemple).

L'assemblage complet d'un dispositif 10 peut aussi être réalisé lors d'un acte médical en procédant successivement à une élévation en température de la feuille 30 en matériau formable ou thermo-formable, un mise en forme de la feuille 30 de matériau formable ou thermo-formable élevée en température par moulage sur un objet ou une partie d'un corps, une perforation de la feuille 30 de matériau formable ou thermo-formable de façon à former une ouverture, et une fixation du guide 50 de positionnement d'un équipement dans ou en regard de l'ouverture réalisée dans la feuille 30. Selon une variante, le guide 50 peut être fixé sur la feuille 30 avant mise en forme de celle-ci.

Avantageusement, et pour mieux se conformer à des formes d'objets ou de parties de corps prédéfinies, la feuille 30 en matériau formable ou thermo-formable peut présenter des épaisseurs et géométries variées.

Ainsi la feuille 30 peut présenter une forme triangulaire, une forme de quadrilatère ou de polygone, une forme circulaire ou ovale ou encore une forme comprenant une partie centrale à partir de laquelle s'étendent une ou plusieurs parties latérales (par exemple un cercle central à partir duquel s'étendent deux bandes).

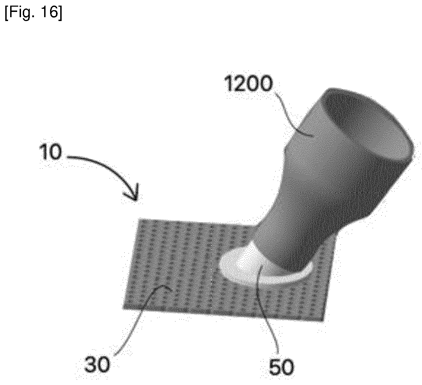

La **figure 16** représente le dispositif d'aide au positionnement de la figure 14 dans lequel est emboitée, par exemple, l'extrémité 1200 d'un outil d'activation d'un distracteur.

Selon un autre exemple, l'extrémité 1200 est l'extrémité d'une sonde à effet Doppler utilise à la visualisation d'une artère et la feuille 30 peut être modelée sur le cou d'une personne aux fins de pouvoir visualiser une partie d'une artère de la personne par des moyens d'imagerie médicale reliés à la sonde à effet Doppler comprenant l'extrémité 1200.

Le dispositif 10 d'aide au positionnement permet de faciliter l'activation externe de distracteurs magnétiques notamment dans le cadre de chirurgie maxillo-faciale, ainsi que dans le cadre de l'orthodontie, et permet notamment un contrôle amélioré de distraction mandibulaire, de distraction maxillaire, de distraction de la voute crânienne postérieure, de distraction symphysaire, de distraction alvéolaire, d'avancée faciale monobloc, de disjonction du palais.

Un tel dispositif trouve également des utilisations dans des actes sur des zones métacarpiennes du corps, pour des opérations de transport osseux.

En d'autres termes, le dispositif 10 d'aide au positionnement comprend la feuille 30 réalisée en matériau formable ou thermo-formable et configurée pour être rapportée sur un sujet et comprend le guide 50 de positionnement de l'élément magnétique de l'outil d'activation assemblé sur la feuille 30.

Le guide de positionnement 50 délimite la cavité 70 de forme prédéterminée configurée pour le positionnement d'une partie d'un l'équipement présentant une forme complémentaire à celle de la cavité 70. La cavité 70 du guide de positionnement 50 peutêtre traversante ou non, selon des variantes.

La cavité 70 du guide 50 peut être de toute forme, par exemple de forme sensiblement cylindrique ou sensiblement tronconique.

Le guide de positionnement 50 est fixé sur la feuille 30 de matériau formable ou thermo-formable par sertissage ou collage, ou encore à l'aide de vis ou de goupilles cylindriques ou coniques.

La cavité 70 peut présenter un axe longitudinal formant un angle de 30 à 90 degrés avec un plan comprenant une base du guide en contact avec la feuille de matériau formable ou thermo-formable, telle que l'embase du demi-guide 50A ou celle du demi-guide 50B, ou encore un plan défini par la forme de la surface de la feuille 30 en contact avec le guide 50.

Le matériau formable ou thermo-formable de la feuille 30 comprend un matériau choisi dans le groupe comprenant : une plaque thermo-formable, du plâtre, du papier mâché, de l'argile, de la résine ou un matériau utilisable pour une impression 3D.

Le guide de positionnement 50 de l'équipement est réalisé dans un matériau choisi dans le groupe comprenant du PEEK, du polyuréthane, du titane, de l'acier inoxydable, de l'or, du plâtre, de la résine, ou du verre.

Le dispositif 10 selon l'invention est configuré pour optimiser le positionnement d'un outil d'activation pour un distracteur à plaques implanté sur un sujet.

La feuille 30 en matériau formable ou thermo-formable peut être agencée dans une version qui présente au moins partiellement la forme d'un visage.

Le dispositif 10 peut être fabriqué par succession d'étapes comprenant :
- une élévation en température de la feuille 30 en matériau formable ou thermo-formable,
- une mise en forme de la feuille 30 de matériau formable ou thermo-formable élevée en température par moulage sur un objet ou une partie du corps d'un sujet,
- une perforation de la feuille 30 de matériau formable ou thermo-formable de façon à former une ouverture, et
- une fixation du guide 50 de positionnement de l'équipement, qui comprend la cavité 70, dans ou en regard de l'ouverture, avant ou après élévation de température et mise en forme de la feuille 30 de matériau formable ou thermo-formable.

L'invention ne se limite bien évidemment pas aux seuls modes de réalisation décrits ci-avant mais à tout ensemble tel que défini dans les revendications.

## Revendications

1. Ensemble (7) d'un appareil d'expansion osseuse (3) et d'un outil d'activation (1) pour un appareil d'expansion osseuse (3), l'outil d'activation (1) comprenant un élément magnétique (130) solidaire d'un axe de rotation (120) de l'outil d'activation (1), l'élément magnétique (130) étant configuré pour interagir coaxialement avec un élément magnétique distant (310) porté par l'appareil d'expansion osseuse (3), l'outil d'activation (1) étant **caractérisé en ce qu'**il comprend en outre un moteur électrique (110) configuré pour opérer une rotation de l'axe de rotation (120), et un module de contrôle (140) de la rotation de l'axe de rotation (120),
l'ensemble (7) comprenant en outre un dispositif (5) d'aide au positionnement de l'élément magnétique (130) de l'outil d'activation (1) par rapport à l'élément magnétique (310) porté par l'appareil d'expansion osseuse (3),
**caractérisé en ce que**
l'appareil d'expansion osseuse (3) est un distracteur à plaques (3); et **en ce que** le dispositif (5) d'aide au positionnement de l'élément magnétique (130) de l'outil d'activation (1) comprend une feuille en matériau formable ou thermo-formable (52) configurée pour être rapportée sur un sujet (9) et comporte un guide de positionnement (54) de l'élément magnétique (130).

2. Ensemble (7) selon la revendication précédente, le module de contrôle (140) étant configuré pour contrôler le sens de rotation de l'axe de rotation (120).

3. Ensemble (7) selon l'une quelconque des revendications précédentes, le module de contrôle (140) étant configuré pour contrôler la vitesse de rotation de l'axe de rotation (120).

4. Ensemble (7) selon l'une quelconque des revendications précédentes, le module de contrôle (140) comprenant des moyens de comptage d'un nombre de portions de tours réalisées lors de la rotation de l'axe de rotation (120).

5. Ensemble (7) selon la revendication précédente, le module de contrôle (140) comprenant des moyens d'arrêt de la rotation de l'axe de rotation (120) lorsque l'axe de rotation (120) a opéré une rotation d'un nombre prédéterminé de tours.

6. Ensemble (7) selon la revendication précédente, le module de contrôle (140) comprenant des moyens de programmation d'un sens de rotation et d'un nombre de tours de l'axe de rotation (120) à opérer selon ce sens de rotation.

7. Ensemble (7) selon l'une quelconque des revendications précédentes, l'outil d'activation (1) comprenant un capteur de champ magnétique (150), configuré pour mesurer une variation de champ magnétique émis par l'élément magnétique distant (310) porté par l'appareil d'expansion osseuse (3) lorsque l'élément magnétique distant (310) est positionné dans le prolongement de l'axe (120) de l'outil d'activation du côté de l'élément magnétique (130) solidaire de l'axe de rotation (120).

8. Ensemble (7) selon la revendication précédente, l'outil d'activation (1) comprenant un module de mesure (150) de champ magnétique configuré pour déterminer une rotation de l'élément magnétique distant (310) d'au moins un nombre de tours prédéterminé autour de l'axe d'écartement de l'appareil d'expansion osseuse (3).

9. Ensemble (7) selon l'une quelconque des revendications précédentes, l'élément magnétique (130) comprenant un aimant ou un ensemble de bobines électromagnétiques configurées pour permettre la génération d'un champ magnétique tournant orienté perpendiculairement à l'axe (120).

## Patentansprüche

1. Anordnung (7) aus einer Knochenexpansionsvorrichtung (3) und einem Aktivierungswerkzeug (1) für eine Knochenexpansionsvorrichtung (3), wobei das Aktivierungswerkzeug (1) ein magnetisches Element (130) aufweist, das fest mit einer Drehachse (120) des Aktivierungswerkzeugs (1) verbunden ist, wobei das magnetische Element (130) konfiguriert ist, um koaxial mit einem entfernten magnetischen Element (310) zu interagieren, das von der Knochenexpansionsvorrichtung (3) getragen wird, wobei das Aktivierungswerkzeug (1) **dadurch gekennzeichnet ist, dass** es ferner einen Elektromotor (110), der so konfiguriert ist, dass er eine Drehung der Drehachse (120) bewirkt, und ein Modul (140) zur Steuerung der Drehung der Drehachse (120) aufweist, wobei die Anordnung (7) außerdem eine Vorrichtung (5) zur Unterstützung der Positionierung des magnetischen Elements (130) des Aktivierungswerkzeugs (1) in Bezug auf das magnetische Element (310) aufweist, das von der Knochenexpansionsvorrichtung (3) getragen wird,
**dadurch gekennzeichnet, dass**
die Knochenexpansionsvorrichtung (3) ein Platten-Distraktor (3) ist; und dass die Vorrichtung (5) zur Unterstützung der Positionierung des magnetischen Elements (130) des Aktivierungswerkzeugs (1) eine Folie aus formbarem oder wärmeformbarem Material (52) aufweist, die so konfiguriert ist, dass sie an einem Subjekt (9) angebracht werden kann, und eine Führung (54) zur Positionierung des magnetischen Elements (130) aufweist.

2. Anordnung (7) nach dem vorhergehenden Anspruch, wobei das Steuermodul (140) konfiguriert ist, die Drehrichtung der Drehachse (120) zu steuern.

3. Anordnung (7) nach einem der vorhergehenden Ansprüche, wobei das Steuermodul (140) konfiguriert ist, die Drehgeschwindigkeit der Drehachse (120) zu steuern.

4. Anordnung (7) nach einem der vorhergehenden Ansprüche, wobei das Steuermodul (140) eine Einrichtung zum Zählen einer Anzahl von Abschnitten von Umdrehungen aufweist, die während der Drehung der Drehachse (120) ausgeführt werden.

5. Anordnung (7) nach dem vorhergehenden Anspruch, wobei das Steuermodul (140) Mittel zum Stoppen der Drehung der Drehachse (120) aufweist, wenn die Drehachse (120) eine Drehung um eine vorbestimmte Anzahl von Umdrehungen vollzogen hat.

6. Anordnung (7) nach dem vorhergehenden Anspruch, wobei das Steuermodul (140) Mittel zur Programmierung einer Drehrichtung und einer Anzahl von Umdrehungen der Drehachse (120) aufweist, die gemäß dieser Drehrichtung zu tätigen sind.

7. Anordnung (7) nach einem der vorhergehenden Ansprüche, wobei das Aktivierungswerkzeug (1) einen Magnetfeldsensor (150) aufweist, der konfiguriert ist, eine Änderung des Magnetfelds zu messen, das von dem von der Knochenexpansionsvorrichtung (3) getragenen entfernten magnetischen Element (310) ausgesendet wird, wenn das entfernte magnetische Element (310) in Verlängerung der Achse (120) des Aktivierungswerkzeugs auf der Seite des magnetischen Elements (130) positioniert ist, die fest mit der Drehachse (120) verbunden ist.

8. Anordnung (7) nach dem vorhergehenden Anspruch, wobei das Aktivierungswerkzeug (1) ein Magnetfeldmessmodul (150) aufweist, das konfiguriert ist, eine Drehung des entfernten magnetischen Elements (310) um mindestens eine vorbestimmte Anzahl von Umdrehungen um die Spreizachse der Knochenexpansionsvorrichtung (3) zu bestimmen.

9. Anordnung (7) nach einem der vorhergehenden Ansprüche, wobei das magnetische Element (130) einen Magneten oder eine Anordnung von elektromagnetischen Spulen aufweist, konfiguriert sind, die Erzeugung eines rotierenden Magnetfelds zu ermöglichen, das senkrecht zu der Achse (120) ausgerichtet ist.

## Claims

1. Assembly (7) of a bone expansion apparatus (3) and an activation tool (1) for a bone expansion apparatus (3), the activation tool (1) comprising a magnetic element (130) solidly attached to a rotation shaft (120) of the activation tool (1), the magnetic element (130) being configured to interact coaxially with a remote magnetic element (310) borne by the bone expansion apparatus (3), the activation tool (1) being **characterised in that** it further comprises an electric motor (110) configured to cause the rotation shaft (120) to rotate, and a control module (140) controlling the rotation of the rotation shaft (120),
wherein the assembly further comprises a device (5) for helping to position the magnetic element (130) of the activation tool (1) with respect to the magnetic element (130) borne by the bone expansion apparatus (3),
**characterised in that** the bone expansion apparatus (3) is a distraction plate apparatus (3); and **in that** the device (5) for helping to position the magnetic element (130) of the activation tool (1) comprises a sheet of formable or thermoformable material (52) configured to be attached to a subject (9) and comprising a guide (54) for positioning the magnetic element (130).

2. Assembly (7) according to the preceding claim, wherein the control module (140) is configured to control a direction of rotation of the rotation shaft (120).

3. Assembly according to any one of the preceding claims, wherein the control module (140) is configured to control the speed of rotation of the rotation shaft (120).

4. Assembly according to any one of the preceding claims, wherein the control module (140) comprises means for counting a number of portions of revolution made during the rotation of the rotation shaft (120).

5. Assembly according to the preceding claim, wherein the control module (140) comprises means for stopping the rotation of the rotation shaft (120) when the rotation shaft (120) has rotated through a predetermined number of revolutions.

6. Assembly according the preceding claim, wherein the control module (140) comprises means for programming a direction of rotation and a number of revolutions of the rotation shaft (120) to be made in this direction of rotation.

7. Assembly according to any one of the preceding claims, wherein the activation tool (1) comprises a magnetic field sensor (150) configured to measure a variation in a magnetic field emitted by the remote magnetic element (310) borne by the bone expansion apparatus (3) when the remote magnetic element (310) is positioned in an extension of the rotation shaft (120) of the activation tool on a side of the magnetic element (130) solidly attached to the rotation shaft (120).

8. Assembly according to the preceding claim, wherein the activation tool (1) comprises a magnetic field measurement module (150) configured to determine a rotation of the remote magnetic element (310) of at least a predetermined number of revolutions about a separation axis of the bone expansion apparatus (3).

9. Assembly according to any one of the preceding claims, wherein the magnetic element (130) comprises a magnet or a set of electromagnetic coils configured to generate a rotating magnetic field oriented perpendicular to the rotation shaft (120).
